# EUROPEAN PATENT SPECIFICATION

(11) **EP 2 044 021 B1**
(45) Date of publication and mention of the grant of the patent: **31.03.2010**
(21) Application number: 07730143.0
(22) Date of filing: 13.06.2007
(51) Int. Cl.: C07D 211/58

(54) **PROCESS FOR PREPARING REMIFENTANIL, INTERMEDIATES THEREOF, USE OF SAID INTERMEDIATES AND PROCESSES FOR THEIR PREPARATION**
VERFAHREN ZUR HERSTELLUNG VON REMIFENTANIL, ZWISCHENPRODUKTE DAVON, VERWENDUNG DIESER ZWISCHENPRODUKTE UND VERFAHREN ZU IHRER HERSTELLUNG
PROCÉDÉ DE PRÉPARATION DE RÉMIFENTANIL, INTERMÉDIAIRES DE CELUI-CI, UTILISATION DESDITS INTERMÉDIAIRES ET PROCÉDÉS POUR LEUR PRÉPARATION

(30) Priority: 15.06.2006 EP 06115527; 15.06.2006 US 804906 P
(43) Date of publication of application: 08.04.2009
(73) Proprietor: Kern Pharma, S.L., 08228 Terrassa (Barcelona) (ES)
(72) Inventor: CERVELLÓ PAGÈS, Jorge, E-08202 Sabadell (ES); CANTÓ VALLVERDÚ, María, E-08190 Sant Cugat Del Vallés (ES)
(74) Representative: ZBM Patents
(86) International application number: PCT/EP2007/055859
(87) International publication number: WO 2007/144391

(56) References cited:
- EP-A1- 0 383 579
- WO-A2-01/40184
- US-A- 3 998 834
- KIRICOJEVIC V.D. ET AL: "An optimized synthesis of a key pharmaceutical intermediate methyl -4-[(1-oxopropyl)phenylamino]piperidine-4- carboxylate" J.SERB.CHEM.SOC., vol. 67, no. 12, 2002, pages 793-802, XP009074906 cited in the application

## Description

The present invention relates to a process for preparing remifentanil, intermediates thereof, use of said intermediates and processes for the preparation thereof.

### BACKGROUND ART

The 4-anilidopiperidine class of opioid analgetics is widely used during surgical procedures as adjuncts to anesthesia. Since the protoptype, fentanyl, **1,** was introduced in the 1960s, several other derivatives (sufentanil, **2**, alfentanil, **3**) have been developed in order to improve its properties. Among them is of special interest remifentanil, because of its ultrashort duration of action. This feature makes it particularly suitable for anesthesia, since provides a more rapid recovery and no accumulation of drug during continuous infusion. 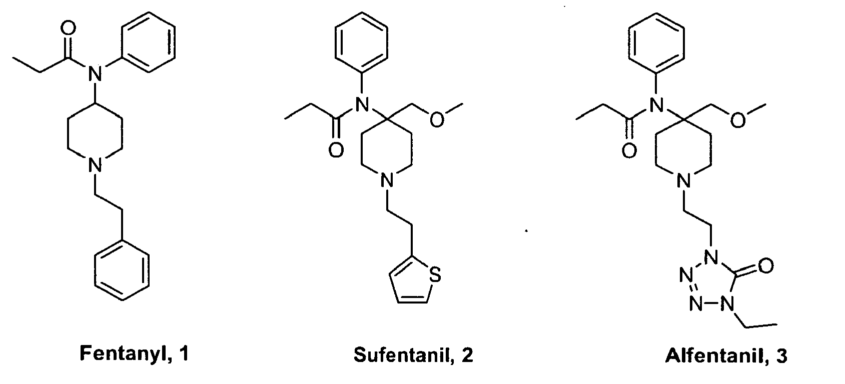 Remifentanil is the INN name of the chemical compound
4-{(methoxycarbonyl)-4-[(1-oxopropyl)phenylamino]-1-piperidine}propanoic acid methyl ester. It has the following chemical formula:

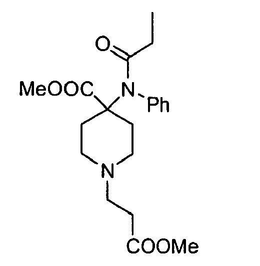

It is currently marketed under the trademark ULTIVA, as its hydrochloride salt form.

Remifentanil was disclosed in the EP383579A patent application. Despite the interesting properties of this compound, few processes for its preparation have been described.

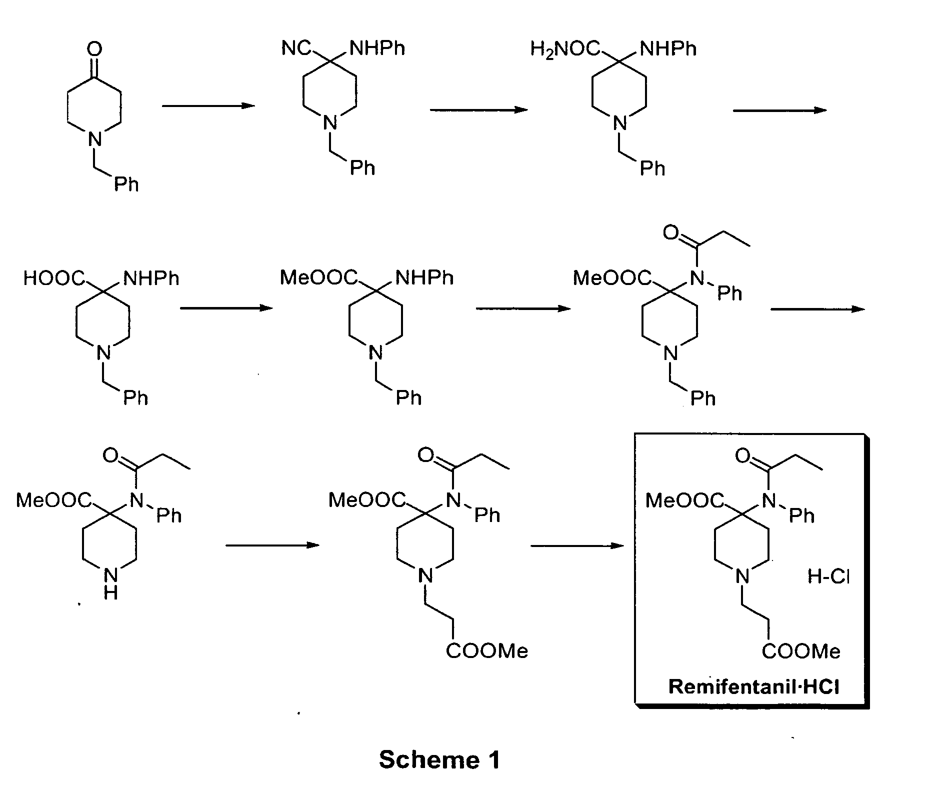

EP383579A discloses generically two processes that may lead to remifentanil: by reaction of a piperidine derivative with a reagent serving to introduce the substituent of the N in the piperidine ring, and by esterifying the carboxy group of the substituent of the N in the piperidine ring. In particular, example 10 of said application describes a process for preparing remifentanil by reaction of the corresponding piperidine with methyl acrylate to render remifentanil as an oil. The hydrochloride salt is prepared by dissolving the free base in methanol and addition of hydrogen chloride **(Scheme 1).**

An optimized synthesis for the preparation of said piperidine intermediate is described in Kiricojevic V.D. et al. J. Serb. Chem. Soc. 67(12)793-802(2002). It starts from 1-benzyl-4-piperidone and comprises seven steps with an 22% overall yield.

International application WO0140184A2 relates to (phenylamino)-4-piperidineanilides as intermediates for the preparation of derivatives and analogs of fentanyl **(Scheme 2).** It also mentions in example 10 B the steps of a possible route of synthesis for preparing remifentanil, using said intermediates, by alkylation of 4-(phenylamino)-4-piperidinecarboxy-(N-methylanilide) with methyl acrylate, conversion of the tertiary amide to methyl ester and reaction with propionyl chloride. It should be noticed that in this approach the starting material is quite far from commercial available precursors.

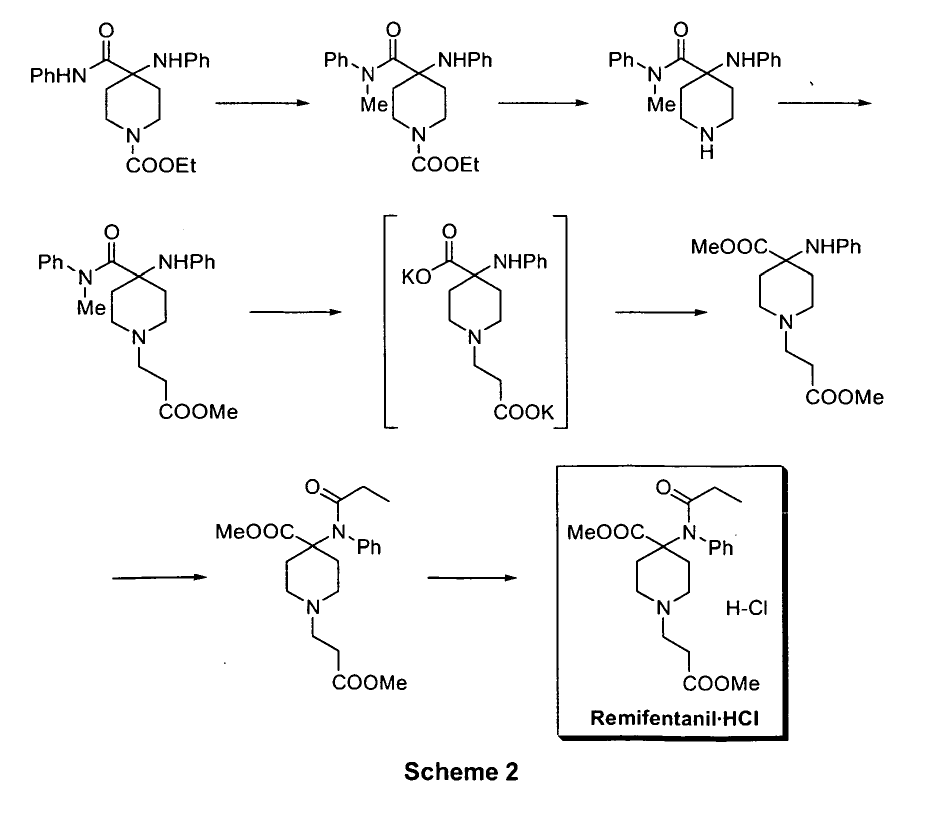

### SUMMARY OF THE INVENTION

The problem to be solved by the present invention is to provide an alternative process for preparing remifentanil. The process is advantageous in several aspects over the processes of the prior art.

Accordingly, a first aspect of the invention relates to a process for preparing a compound of formula (I) or pharmaceutically acceptable salts thereof 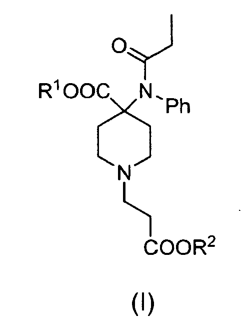 wherein R¹ and R² are independently a C₁-C₄ linear or branched alkyl or H, which comprises converting the nitrile group of a compound of formula (II) 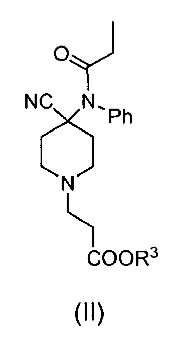 wherein R³ is a C₁-C₄ linear or branched alkyl or H,
into a -COOR¹ group, wherein R¹ is as defined above, in an appropriate solvent system, and optionally, when in the resulting compound of formula (I) at least one of R¹ and R² are not a methyl group transforming said R to a methyl group, and optionally, if desired, further converting the compound of formula (I) or a salt thereof into a pharmaceutically acceptable salt thereof.

A second aspect of the invention relates to a compound of formula (II): 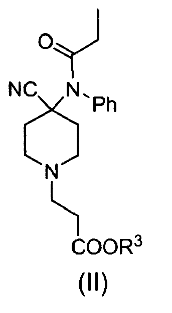 wherein R³ is as defined above.

A third aspect of the invention relates to a compound of formula (III): 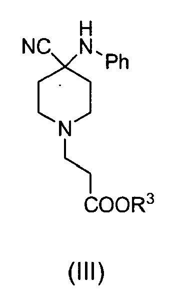 wherein R³ is as defined above.

A fourth aspect of the invention relates to the use of a compound of formula (II) as defined in the second aspect of the invention and its corresponding embodiments, for the preparation of remifentanil

A fifth aspect of the invention relates to the use of a compound of formula (III) as defined in the third aspect of the invention and its corresponding embodiments, for the preparation of remifentanil.

A sixth aspect of the invention relates to a process for preparing a compound of formula (II) as defined in the second aspect of the invention and its corresponding embodiments, which comprises the acylation of a compound of formula (III) 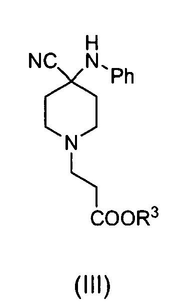 wherein R³ is as defined above,
with an acylating agent able to introduce a propionyl group.

A seventh aspect of the invention relates to a process for preparing a compound of formula (III) as defined in the third aspect of the invention and its corresponding embodiments, which comprises the reaction of 3-(4-oxo-1-piperidine)propanoic acid, C₁-C₄ alkyl ester with aniline and a source of cyanide, under Strecker type reaction conditions.

The process of the present invention presents several advantages that are highly desirable for the manufacture of remifentanil on an industrial scale. It provides an alternative process, wherein the number of steps for preparing remifentanil from commercial products is significantly reduced compared to the processes known in the art, the starting products are readily available, the process is economically advantageous and renders remifentanil in a highly simplified and efficient manner.

Prior numerous attempts described in the literature failed to reduce the number of steps of remifentanil synthesis (e.g. by unsuccessfully trying the direct conversion of an anilino-nitrile intermediate to an anilino-ester intermediate) (Kiricojevic V.D. et al. J.Serb.Chem.Soc. 67(12)793-802(2002)). One embodiment of the invention not only reduces the number of steps of the synthesis route but also succeeds in the direct conversion of the nitrile group to the ester group. Thus, remifentanil and even its hydrochloride salt may be obtained in one step from the nitrile intermediate, which may be easily obtained from simple and cheap starting materials.

The intermediates provided by the present invention allow the preparation of remifentanil in very few steps compared to the processes known in the art.

### Definitions

By C₁-C₄ alkyl or C₁-C₄linear or branched alkyl, as used herein, it is understood a linear or branched alkyl group which contains up to 4 carbon atoms. Thus it includes a methyl, ethyl, propyl, isopropyl, butyl, isobutyl, sec-butyl and tert-butyl group.

By C₂-C₄ linear or branched alkyl, as used herein, it is understood a linear or branched alkyl group which contains from 2 to 4 carbon atoms. Thus it includes a ethyl, propyl, isopropyl, butyl, isobutyl, sec-butyl and *tert*-butyl group.

### DETAILED DESCRIPTION OF PARTICULAR EMBODIMENTS

As said above, in the process according to the first aspect of the invention the nitrile group of a compound of formula (II) is converted into a -COOR¹ group, wherein R¹ is a C₁-C₄ linear or branched alkyl or H.

The best conditions to carry out the process vary according to the parameters considered by the person skilled in the art, such as the R groups, the solvents, temperature and similar. Such reaction conditions may be easily determined by the person skilled in the art by routine tests, and with the teaching of the examples included in this document.

The nitrile group may be converted to a -COOH group by hydrolysis in an acid or basic medium. Suitable acidic conditions include strong acids such as hydrochloric acid, sulphuric acid or trifluoroacetic acid in polar solvents such as water or acetic acid. Suitable basic conditions include sodium or potassium hydroxide as bases and water or alcohols as solvents. Bacteria can also be used for this transformation. Typical details of this process embodiment can be found in Larock, R. C. Comprehensive Organic Transformation, 2nd Ed, Wiley-VCH, p.1986. The nitrile group may be converted to a -COOR¹ group wherein R¹ means C₁-C₄ linear or branched alkyl, by an alcoholysis reaction. Typical details of this process embodiment can be found in Larock, R. C. Comprehensive Organic Transformation, 2nd Ed, Wiley-VCH, p.1987.

As said in the first aspect of the invention, when in the compound resulting from the conversion of the nitrile group to a COOR¹ group at least one of R¹ and R² are not a methyl group, the conversion is optionally followed by a transformation of said R to a methyl group. Said transformation of said R may be effected by an esterification reaction, when said R is H, or by a transesterification reaction when said R is a C₂-C₄ linear or branched alkyl. Said esterification reaction may be carried out with a hydrogen chloride solution in methanol e.g. as described in Synth. Commun. 1998, 28, 471-474. Said transesterification reaction may also be carried out with a hydrogen chloride solution in methanol e.g. as the described in Synth. Commun. 1998, 28, 471-474.

In a preferred embodiment, the nitrile group is converted to a -COOR¹ group, wherein R¹ means a C₁-C₄ linear or branched alkyl, by an alcoholysis reaction. Therefore, the solvent system for carrying out said conversion preferably comprises a C₁-C₄ linear or branched alcohol and an organic or inorganic strong acid. The strong acid is preferably selected from the group consisting of hydrochloric acid, hydrobromic acid, methanosulfonic acid and sulfuric acid.

The alcoholysis reaction is preferably carried out at a temperature comprised in the range from 80 °C to 0 °C. More preferably from 40 °C to 10 °C, and yet more preferably at room temperature.

Preferably the alcoholysis is a methanolysis or an ethanolysis. Therefore, the alcohol is preferably selected from the group consisting of methanol and ethanol, more preferably methanol. When methanol is employed, the nitrile is directly converted to a COOMe group. When ethanol is employed the nitrile group is converted to a COOEt group.

In one embodiment, the process comprises the conversion of the nitrile group of a compound of formula (II) wherein R³ is a ethyl group, into a COOEt group in ethanol and in the presence of a strong organic or inorganic acid and the ethyl ester group(s) of the compound obtained are further transformed to methyl ester by transesterification reaction in methanol.

In a preferred embodiment, R¹, R² and R³ are a methyl group. In a particularly preferred embodiment, R¹, R² and R³ are a methyl group, the alcoholysis reaction is a methanolysis and is carried out in methanol and in the presence of an organic or inorganic strong acid. In a yet more particularly preferred embodiment, the strong acid is hydrochloric acid, advantageously in this case, because remifentanil hydrochloride salt is directly obtained.

The remifentanil hydrochloride salt thus obtained may be further purified if necessary, by recrystallization. Alternatively, it may be purified by conversion of the salt to the free base and again conversion to the desired pharmaceutical salt.

Compounds of formula (I) obtained by the process of the present invention may be converted into pharmaceutically acceptable salts, and salts may be converted into free compounds, by conventional methods.

Generally, the meaning of R² in the compound of formula (I) obtained will be the same as the meaning of R³ in the intermediate of formula (II) employed. However, in some cases it may be different, since, as would be well-known to the skilled in the art, depending on the reaction conditions employed for the transformation of the nitrile group of the intermediate of formula (II), the group COOR³ may suffer a transesterification reaction (e.g. when the conversion of the nitrile group is done by means of an alcoholysis reaction and the alcohol employed is R²-OH, wherein R² is different from R³ and in the compound of formula (I) R¹=R²) or a hydrolysis reaction (e.g. when the conversion of the nitrile group is done by means of a hydrolysis reaction and R³ does not mean H). In a preferred embodiment, R² in the compound of formula (I) obtained has the same meaning as R³ in the intermediate of formula (II) employed.

Preferably, the intermediate of formula (II) is prepared by acylation of a compound of formula (III) 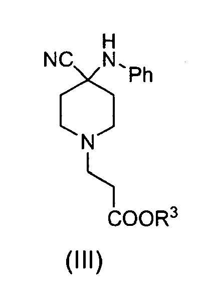 with an acylating agent able to introduce a propionyl group.

Preferably R³ is methyl.

Suitable acylating agents include propionyl halides, such as propionyl chloride, propionyl bromide, anhydrides such as propionic anhydride, including also mixed or fluorated anhydrides containing a propionyl moiety. Preferably, the acylating agent is selected from the group consisting of propionyl chloride, propionyl bromide and propionic anhydride. More preferably, the acylating agent is propionyl chloride.

The acylation reaction for preparing the compound of formula (II) may be carried out in a variety of solvents. Preferably, when the acylating agent is not an anhydride, the solvent is a polar aprotic solvent. More preferably, the solvent is selected from the group consisting of dichloroethane, tetrahydrofuran, dimethoxyethane and toluene. Particularly good results are obtained when it is carried out in toluene. When the acylating reagent is an anhydride, propionic acid can be used also as solvent. The reaction can be carried out in the presence of an acylation catalyst, such as dimethylaminopyridine. The reaction is preferably carried out at a temperature comprised in the range from room temperature to 100 °C. More preferably from 45 °C to 90 °C, and yet more preferably at 85 °C.

Preferably, the intermediate of formula (III) is prepared by condensation of 3-(4-oxo-1-piperidine)propanoic acid, C₁-C₄ alkyl ester with aniline and an a source of cyanide, under Strecker type reaction conditions. Suitable source of cyanide include organic and inorganic cyanides, such as ammonium cyanide, trimethylsilyl cyanide, sodium cyanide, and potassium cyanide. Preferably, the source of cyanide is sodium cyanide.

The condensation reaction with aniline to render the compound of formula (III) may be carried out in a variety of solvents. Suitable solvents are alcohols, acetic acid and chlorinated solvents, preferably selected from the group consisting of C₁-C₄ linear or branched alcohols, such as methanol, ethanol, 2-propanol, 1-propanol, and dichloromethane. Preferably, it is carried out in methanol, in order to avoid transesterification by-products.

The condensation reaction with aniline is preferably carried out in the presence of an acid catalyst, such as acetic acid, trifluoroacetic acid, aqueous hydrochloric acid, p-toluenesulfonic acid or methanesulfonic acid. More preferably, the acid catalyst is acetic acid. Particularly good results were obtained employing methanol as a solvent and acetic acid as catalyst.

The reaction may be carried out at a temperature range from 0 °C to 40 °C, preferably at room temperature. In a preferred embodiment the temperature is subsequently increased to a temperature comprised in the range from 40 °C to 70 °C.

The 3-(4-oxo-1-piperidine)propanoic acid, C₁-C₄ alkyl ester may be prepared in different ways, preferably by way of a conjugated addition reaction between 4-piperidone and the corresponding C₁-C₄ alkyl acrylate. The methyl or ethyl ester are preferred, most preferred is the methyl ester. The reaction may be carried out in an inert organic solvent, such as acetonitrile, a C₁-C₄ linear or branched alcohol, e.g. methanol or ethanol, an ether e.g. diethyl ether, dioxane, and an aromatic hydrocarbon, e.g. benzene, toluene and more preferably in methanol.

Preferred compounds of formula (II) are those wherein R³ is methyl or ethyl. Yet more preferred compounds of formula (II) are those wherein R³ is methyl.

Preferred compounds of formula (III) are those wherein R³ is methyl or ethyl. Yet more preferred compounds of formula (III) are those wherein R³ is methyl.

Throughout the description and claims the word "comprise" and variations of the word, such as "comprising", is not intended to exclude other technical features, additives, components, or steps.

Additional objects, advantages and features of the invention will become apparent to those skilled in the art upon examination of the description or may be learned by practice of the invention. The following examples are provided by way of illustration, and is not intended to be limiting of the present invention.

### EXAMPLES

### Example 1 : 3-(4-oxo-1-piperidine)propanoic acid, methyl ester

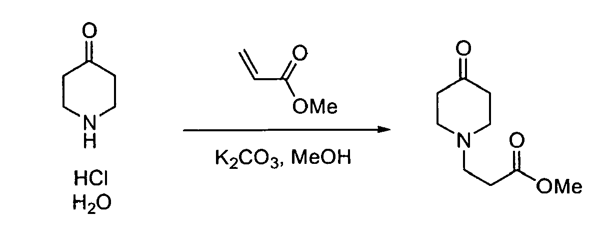

To a suspension of 4-piperidone hydrate hydrochloride (125 g, 0.81 mol) and methyl acrilate (96 ml, 1.07 mol) in methanol (800 mL) is added potassium carbonate (169 g, 1.22 mol) at room temperature. The suspension is stirred at room temperature for 4 hours. The suspension is then filtered and the filtrate concentrated to a residue. The residue is dissolved in water (170 ml) and extracted with ethyl acetate (1L). The layers are separated and the aqueous layer is extracted again with ethyl acetate (2x250 ml). The combined organic phases are dried over anhydrous sodium sulphate and concentrated to give 3-(4-oxo-1-piperidine)propanoic acid, methyl ester (133.3 g, 88%) as an oil that solidifies on standing.

| Elemental Analysis for C₉H₁₅NO₃ | | | |
|---|---|---|---|
| | %C | %H | %N |
| Found: | 58.30 | 8.18 | 7.48 |
| Calculated: | 58.36 | 8.16 | 7.56 |

### Example 2 : 3-(4-cyano-4-phenylamino-1-piperidine)propanoic acid, methyl ester

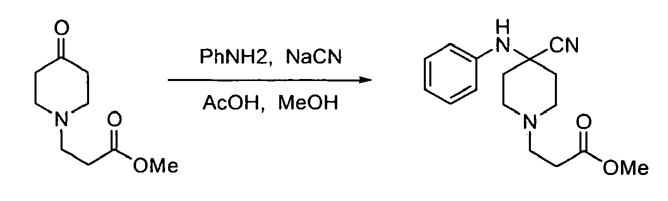

To a stirred mixture of 3-(-4-oxo-1-piperidine)propanoic acid, methyl ester (180 g, 0.97 mol), aniline (143 ml, 1.57 mol) and acetic acid (145 ml, 2.54 mol) in methanol (900 ml) is added dropwise a solution of sodium cyanide (50 g, 1.02 mol) in water (160 ml) at room temperature over a thirty minutes period. The mixture is stirred at 60 °C for four hours. Then the mixture is cooled to 0°C and basified to pH 10-11 with sodium hydroxide 33% while a white precipitated is formed. 350 ml of water are added and the mixture is allowed to stir at 0 °C overn ight.

The mixture is then filtered with suction and the ^{.}solid washed with a mixture 1:1 of water and methanol to yield 3-(4-phenylamino-4-cyano-1-piperidine)propanoic acid methyl ester (124.8 g, 45%) as a white solid; m.p. 96-100 °C.
**¹H-NMR** (250MHz, CDCl₃): δ 7.24 (t, 2H), 6.91 (m, 3H), 3.68 (s, 3H), 2.88-2.70 (m, 2H), 2.74 (t, 2H), 2.49 (t, 2H), 2.54-2.40 (m, 2H), 2.32 (d, 2H), 1.89 (ddd, 2H). **¹³C-NMR (62.5 MHz, CDCl₃):** δ 172.5, 143.0, 129.0, 120.7, 120.3, 117.6, 52.9, 52.7, 51.4, 48.8, 35.8, 32.1.

| Elemental Analysis for C₁₆H₂₁N₃O₂ | | | |
|---|---|---|---|
| | %C | %H | %N |
| Found: | 67.02 | 7.43 | 14.75 |
| Calculated: | 66.88 | 7.37 | 14.62 |

### Example 3 : 3-[4-cyano-4-[(1-oxopropyl)phenylamino)]-1-piperidine]propanoic acid, methyl ester

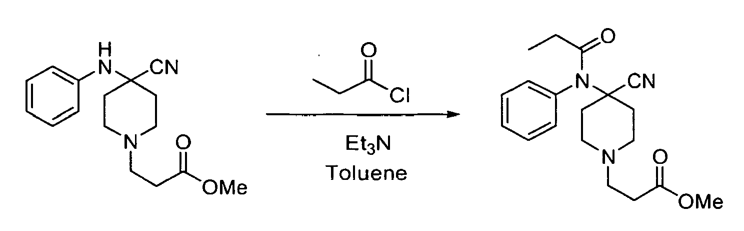

Propionyl chloride (9.5 ml, 108.73 mmol) is added to a stirred solution of 3-(4-phenylamino-4-cyano-1-piperidine)propanoic acid methyl ester (10 g, 34.80 mmol) in toluene (100 ml). The mixture is refluxed for 3 hours, then triethylamine (5 ml, 35.92 mmol) is added gradually over 1 h and the stirring continued overnight. The mixture is cooled to room temperature and the contents are poured into a 25% potassium carbonate solution (100 ml).

The layers are separated and the aqueous layer is extracted with toluene (2x60 ml). The combined organic layers are dried over magnesium sulphate, filtered and concentrated under vacuum to yield 3-[4-cyano-4-[(1-oxopropyl)phenylamino)]-1-piperidine)propanoic acid, methyl ester (11.9 g, 99%) as an oil, which is used in the next reaction without further purification.
**¹H-NMR** (250MHz, CDCl₃): δ 7.46-7.38 (m, 3H,), 7.20-7.11 (m, 2H), 3.63 (s, 3H), 2.83 (d, J=12.6 Hz, 2H), 2.67 (t, J=7.24 Hz, 2H), 2.42 (t, J=7.24 Hz, 2H), 2.50-2.28 (m, 4H), 1.91 (q, J=7.34 Hz, 2H), 1.54 (2t, J≈J'=12,47 Hz, J"=3.40 Hz, 2H), 0.99 (t, J=7.3 Hz, 3H). **¹³C-NMR (62.5 MHz, CDCl₃):** δ 174.2, 172.6, 138.3, 130.3, 129.7, 129.3, 119.2, 56.1, 52.9, 51.6, 49.7, 34.9, 32.1, 29.5, 8.9.

### Example 4 : 3-[4-methoxycarbonyl-4-[(1-oxopropyl)phenylamino)]-1-piperidine]propanoic acid, methyl ester, hydrochloride

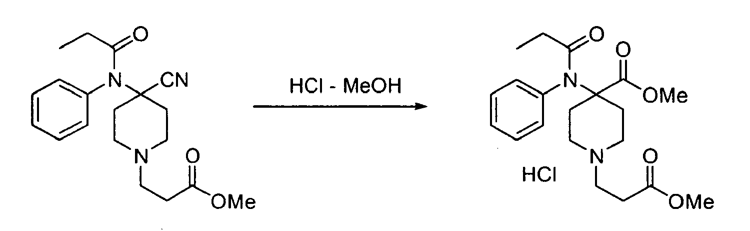

3-[4-cyano-4-[(1-oxopropyl)phenylamino)]-1-piperidine)propanoic acid, methyl ester (10.5 g, 30.66 mmol) is dissolved in a 3.4 M solution of hydrogen chloride in methanol (72 ml) and the solution stirred at room temperature for 24 hours while a white precipitated is formed. The mixture is then cooled to 0 °C and the stirring continued for 3 hours. The mixture is filtered to yield 3-[4-methoxycarbonyl-4-[(1-oxopropyl)phenylamino)]-1-piperidine]propanoic acid methyl ester hydrochloride (7.8 g, 62 %) as a white solid.

The hydrochloride salt is recrystallised heating with methyl ethyl ketone and adding methanol while heating until the solid goes back into solution. Upon cooling the salt precipitates as a white solid.

### Example 5 : 3-[4-methoxycarbonyl-4-[(1-oxopropyl)phenylamino)]-1-piperidine]propanoic acid, methyl ester

3-[4-cyano-4-[(1-oxopropyl)phenylamino)]-1-piperidine)propanoic acid, methyl ester (17.31 g, 50.40 mmol) is dissolved in a 4.0 M solution of hydrogen chloride in methanol (100 ml) and the solution stirred at room temperature for 24 hours while a white precipitated is formed. The mixture is then cooled to 0 °C and the stirring continued for 3 hours. The mixture is filtered to yield 3-[4-methoxycarbonyl-4-[(1-oxopropyl)phenylamino)]-1-piperidine]propanoic acid methyl ester hydrochloride (13.40 g, 64%) as a white solid.

This solid is dissolved in water (130 ml), then toluene (75 ml) is added and the mixture basified with a 25% potassium carbonate solution to pH =9. The layers are separated and the aqueous layer is extracted twice with toluene (60 ml). The combined organic layers are dried over anhydrous sodium sulphate, filtered and concentrated under vacuum to yield 3-[4-methoxycarbonyl-4-[(1-oxopropyl)phenylamino)]-1-piperidine]propanoic acid methyl ester (11.21 g, 60%) as an oil.

The HCl salt is obtained by dissolving the free base oil in methanol and adding an HCl solution in methanol. The solution is then cooled to complete crystallisation, and the solid filtered to yield 3-[4-methoxycarbonyl-4-[(1-oxopropyl)phenylamino)]-1-piperidine]propanoic acid methyl ester hydrochloride (9.75 g, 47%) as a white solid in a pharmaceutical grade. m.p. 188-189 °C

| Elemental Analysis for C₂₀H₂₉ClN₂O₅ | | | |
|---|---|---|---|
| | %C | %H | %N |
| Found: | 58.23 | 7.05 | 6.68 |
| Calculated: | 58.18 | 7.08 | 6.78 |

## Claims

1. A process for preparing a compound of formula (I) or pharmaceutically acceptable salts thereof 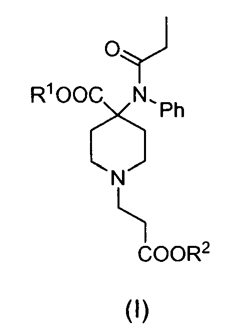 wherein R¹ and R² are independently a C₁-C₄ linear or branched alkyl or H, which comprises converting the nitrile group of a compound of formula (II) 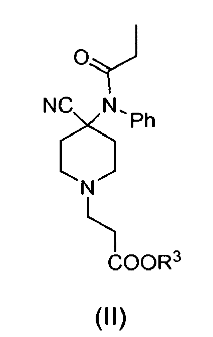 wherein R³ is a C₁-C₄ linear or branched alkyl or H,
directly into a -COOR¹ group, wherein R¹ is as defined above, in an appropriate solvent system, and optionally, when in the resulting compound of formula (I) at least one of R¹ and R² are not a methyl group transforming said R to a methyl group, and optionally, if desired, further converting the compound of formula (I) or a salt thereof into a pharmaceutically acceptable salt thereof.

2. The process according to claim 1, wherein said solvent system comprises a C₁-C₄ alcohol and a strong organic or inorganic acid selected from the group consisting of hydrochloric acid, hydrobromic acid, methanosulfonic acid and sulfuric acid.

3. The process according to claim 1, wherein R¹, R²and R³ are a methyl group.

4. The process according to claim 3, wherein said solvent system comprises methanol and a strong organic or inorganic acid.

5. The process according to claim 4, wherein said acid is hydrochloric acid.

6. The process according to any of the preceding claims, further comprising the step of preparing the compound of formula (II) by acylation of a compound of formula (III) 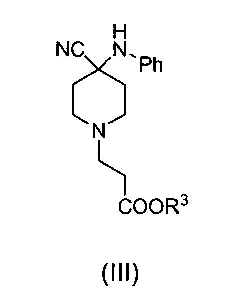 wherein R³ is as defined above, with an acylating agent able to introduce a propionyl group.

7. The process according to claim 6, further comprising the step of preparing the compound of formula (III) by reaction of 3-(4-oxo-1-piperidine)propanoic acid, C₁-C₄ alkyl ester with aniline and a source of cyanide, under Strecker type reaction conditions.

8. A compound of formula (II): 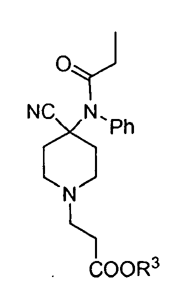 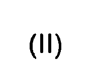 wherein R³ is as defined above.

9. A compound according to claim 8, wherein R³ is methyl or ethyl.

10. A compound according to claim 9, wherein R³ is methyl.

11. A compound of formula (III): 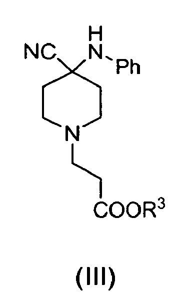 wherein R³ is as defined above.

12. Use of a compound of formula (II) as defined in any of claims 8 to 10, for the preparation of remifentanil.

13. Use of a compound of formula (III) as defined in claim 11, for the preparation of remifentanil.

14. A process for preparing a compound of formula (II) as defined in any of claims 8 to 10, which comprises the acylation of a compound of formula (III) 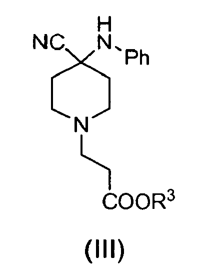 wherein R³ is as defined above,
with an acylating agent able to introduce a propionyl group.

15. A process for preparing a compound of formula (III) as defined in claim 11, which comprises the reaction of 3-(4-oxo-1-piperidine)propanoic acid, C₁-C₄ alkyl ester with aniline and a source of cyanide, under Strecker type reaction conditions.

## Patentansprüche

1. Verfahren zur Herstellung einer Verbindung der Formel (I) oder pharmazeutisch verträglicher Salze davon 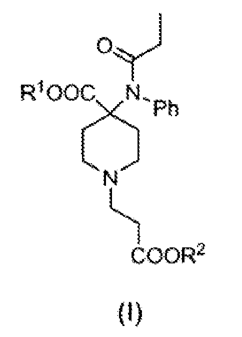 worin R¹ und R² unabhängig voneinander ein unverzweigtes oder verzweigtes C₁-C₄-Alkyl oder H sind, bei dem die Nitrilgruppe einer Verbindung der Formel (II) 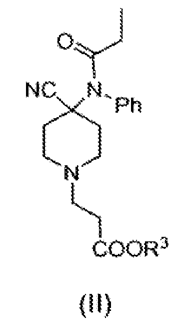 worin R³ ein unverzweigtes oder verzweigtes C₁-C₄-Alkyl oder H ist,
in einem geeigneten Lösungsmittelsystem direkt zu einer -CCOR¹-Gruppe reagiert, worin R¹ der obenstehenden Definition entspricht, und bei dem gegebenenfalls, wenn in der daraus entstandenen Verbindung der Formel (I) mindestens einer der Reste R¹ und R² nicht eine Methylgrupe ist, das besagte R in eine Methylgruppe überführt wird, und bei dem gegebenenfalls, wenn gewünscht, die Verbindung der Formel (I) oder ein Salz derselben weiterhin in ein pharmazeutisch verträgliches Salz davon verwandelt wird.

2. Verfahren nach Anspruch 1, bei dem das besagte Lösungsmittelsystem einen C₁-C₄-Alkohol und eine starke organische oder anorganische Säure umfasst, die aus der Gruppe bestehend aus Salzsäure, Bromwasserstoffsäure, Methansulfonsäure und Schwefelsäure ausgewählt wird.

3. Verfahren nach Anspruch 1, worin R¹, R² und R³ eine Methylgruppe sind.

4. Verfahren nach Anspruch 3, bei dem das besagte Lösungsmittelsystem Methanol und eine starke organische oder anorganische Säure umfasst.

5. Verfahren nach Anspruch 4, bei dem die besagte Säure Salzsäure ist.

6. Verfahren nach einem der vorstehenden Ansprüche, das weiterhin den Verfahrensschritt umfasst, bei dem eine Verbindung der Formel (II) durch Acylierung einer Verbindung der Formel (III) 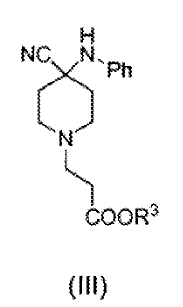 worin R³ der obenstehenden Definition entspricht, mithilfe eines Acylierungsreagens, das in der Lage ist, eine Propionylgruppe einzubringen, hergestellt wird.

7. Verfahren nach Anspruch 6, das weiterhin den Verfahrensschritt umfasst, bei dem die Verbindung der Formel (III) durch Reaktion von 3-(4-oxo-1-piperidin)Propansäure, C₁-C₄-Alkylester mit Anilin und einer Cyanidquelle unter den Bedingungen einer Strecker-artigen Reaktion hergestellt wird.

8. Verbindung der Formel (II): 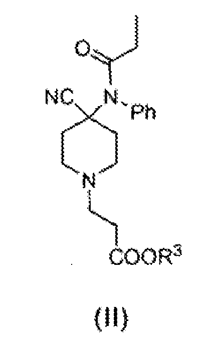 worin R³ der obenstehenden Definition entspricht.

9. Verbindung nach Anspruch 8, worin R³ Methyl oder Ethyl ist.

10. Verbindung nach Anspruch 9, worin R³ Methyl ist.

11. Verbindung der Formel (III): 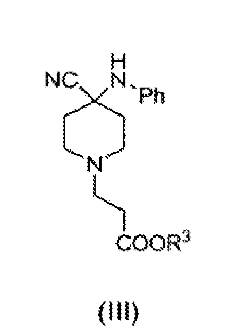 worin R³ der obenstehenden Definition entspricht.

12. Verwendung einer Verbindung der Formel (II), wie sie in einem der Ansprüche 8 bis 10 definiert wird, für die Herstellung von Remifentanil.

13. Verwendung einer Verbindung der Formel (III), wie sie in Anspruch 11 definiert wird, für die Herstellung von Remifentanil.

14. Verfahren zur Herstellung einer Verbindung der Formel (II), wie sie in einem der Ansprüche 8 bis 10 definiert wird, bei dem eine Verbindung der Formel (III) 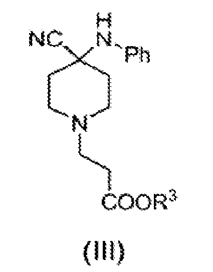 worin R³ der obenstehenden Definition entspricht,
mithilfe eines Acylierungsreagens, das in der Lage ist, eine Propionylgruppe einzubringen, acyliert wird.

15. Verfahren zur Herstellung einer Verbindung der Formel (III) gemäß der Definition in Anspruch 11, das die Reaktion von 3-(4-oxo-1-piperidin)Propansäure, C₁-C₄-Alkylester mit Anilin und einer Cyanidquelle unter den Bedingungen einer Strecker-artigen Reaktion umfasst.

## Revendications

1. Procédé pour préparer un composé de formule (I) ou des sels acceptables sur le plan pharmaceutique de celui-ci 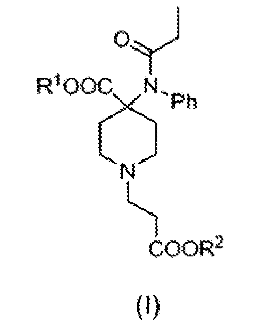 dans laquelle formule R¹ et R² sont indépendamment un alkyle linéaire ou ramifié en C₁ à C₄ ou H, lequel procédé comprend la conversion du groupe nitrile d'un composé de formule (II) 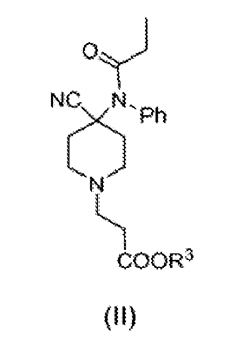 dans laquelle R³ est un alkyle linéaire ou ramifié en C₁ à C₄ ou H, directement en un groupe -COOR¹, dans lequel R¹ est tel que défini précédemment, dans un système de solvant approprié, et éventuellement, lorsque dans le composé de formule (I) résultant au moins l'un parmi R¹ et R² n'est pas un groupe méthyle la transformation dudit R en un groupe méthyle, et éventuellement, si désiré, une conversion ultérieure du composé de formule (I) ou un sel de celui-ci en un sel acceptable sur le plan pharmaceutique de celui-ci.

2. Procédé selon la revendication 1, dans lequel ledit système de solvant comprend un alcool en C₁ à C₄ et un acide fort organique ou inorganique choisi dans le groupe constitué de l'acide chlorhydrique, l'acide bromhydrique, l'acide méthanosulfonique et l'acide sulfurique.

3. Procédé selon la revendication 1, dans lequel R¹, R² et R³ sont un groupe méthyle.

4. Procédé selon la revendication 3, dans lequel ledit système de solvant comprend du méthanol et un acide fort organique ou inorganique.

5. Procédé selon la revendication 4, dans lequel ledit acide est l'acide chlorhydrique.

6. Procédé selon l'une quelconque des revendications précédentes, comprenant en outre l'étape consistant à préparer le composé de formule (II) par acylation d'un composé de formule (III) 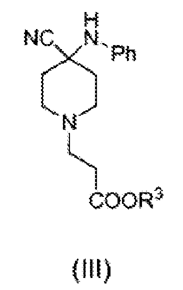 dans laquelle R³ est tel que défini précédemment, avec un agent d'acylation capable d'introduire un groupe propionyle.

7. Procédé selon la revendication 6, comprenant en outre l'étape consistant à préparer le composé de formule (III) par une réaction d'un ester d'alkyle en C₁ à C₄ d'acide 3-(4-oxo-1-pipéridine)propanoïque avec de l'aniline et une source de cyanure, dans des conditions de réaction de type Strecker.

8. Composé de formule (II) : 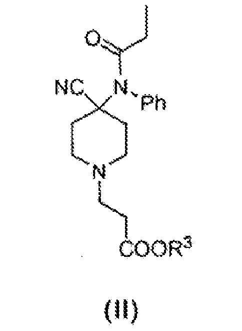 dans laquelle formule R³ est tel que défini précédemment.

9. Composé selon la revendication 8, dans lequel R³ est un méthyle ou un éthyle.

10. Composé selon la revendication 9, dans lequel R³ est un méthyle.

11. Composé de formule (III) : 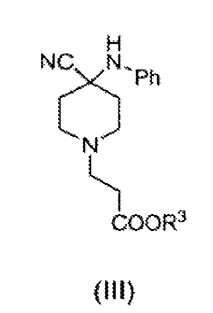 dans laquelle formule R³ est tel que défini précédemment.

12. Utilisation d'un composé de formule (II) tel que défini dans l'une quelconque des revendications 8 à 10, pour la préparation de rémifentanil.

13. Utilisation d'un composé de formule (III) tel que défini dans la revendication 11, pour la préparation de rémifentanil.

14. Procédé pour préparer un composé de formule (II) tel que défini dans l'une quelconque des revendications 8 à 10, qui comprend l'acylation d'un composé de formule (III) 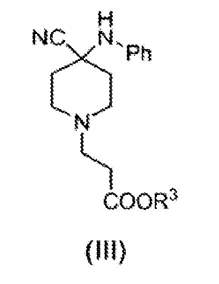 dans laquelle formule R³ est tel que défini précédemment,
avec un agent d'acylation capable d'introduire un groupe propionyle.

15. Procédé pour préparer un composé de formule (III) tel que défini dans la revendication 11, qui comprend la réaction d'un ester d'alkyle en C₁ à C₄ d'acide 3-(4-oxo-1-pipéridine)propanoïque avec de l'aniline et une source de cyanure, dans des conditions de réaction de type Strecker.
